# EUROPÄISCHE PATENTSCHRIFT

(11) **EP 1 711 826 B1**
(45) Veröffentlichungstag und Bekanntmachung des Hinweises auf die Patenterteilung: **28.10.2009**
(21) Anmeldenummer: 05707861.0
(22) Anmeldetag: 27.01.2005
(51) Int. Cl.: G01N 33/543

(54) **BIOSENSOR UND VERFAHREN ZU DESSEN BETRIEB**
BIOSENSOR AND METHOD FOR OPERATING THE LATTER
BIOCAPTEUR ET PROCEDE POUR LE FAIRE FONCTIONNER

(30) Priorität: 05.02.2004 DE 102004005710
(43) Veröffentlichungstag der Anmeldung: 18.10.2006
(73) Patentinhaber: SIEMENS AKTIENGESELLSCHAFT, 80333 München (DE)
(72) Erfinder: SIMON, Elfriede, 80639 München (DE); FLEISCHER, Maximilian, 85635 Höhenkirchen (DE); MEIXNER, Hans, 85540 Haar (DE); HAINDL, Corinna, 68163 Mannheim (DE)
(86) Internationale Anmeldenummer: PCT/EP2005/050343
(87) Internationale Veröffentlichungsnummer: WO 2005/075994

(56) Entgegenhaltungen:
- WO-A-00/62048
- GB-A- 2 276 724
- US-A- 4 778 751
- US-A- 5 945 294
- US-A1- 2005 176 067
- US-B1- 6 673 533
- NIWA O ET AL: "ELECTROCHEMICAL BEHAVIOR OF REVERSIBLE REDOX SPECIES AT INTERDIGITATED ARRAY ELECTRODES WITH DIFFERENT GEOMETRIES: CONSIDERATION OF REDOX CYCLING AND COLLECTION EFFICIENCY" ANALYTICAL CHEMISTRY, AMERICAN CHEMICAL SOCIETY. COLUMBUS, US, Bd. 62, Nr. 5, 1990, Seiten 447-452, XP000943524 ISSN: 0003-2700
- KREUZER M P ET AL: "Development of an immunosensor for the determination of allergy antibody (IgE) in blood samples" ANALYTICA CHIMICA ACTA, Bd. 442, Nr. 1, 31. August 2001 (2001-08-31), Seiten 45-53, XP002347367 ISSN: 0003-2670

## Beschreibung

Die Erfindung betrifft einen Biosensor der mit einer Beschichtung zum Nachweis von Antigen/Antikörper-Reaktionen ausgestattet ist.

In der modernen Analytik und vor allem in der modernen klinischen Diagnostik wird die Nachfrage nach einfachen und schnellen Testmethoden, die dazu sehr kostengünstig sind, immer größer. Insbesondere Systeme die eine große Anzahl von Analysen in kurzer Zeit bestimmen können, wie beispielsweise high throughput screening Methoden, z.B bei der Arzneimittelproduktion, Umwelt- oder Lebensmittelanalytik, oder viele verschiedene Analyten (z.B. mittels Sensorarrays) annähernd gleichzeitig untersuchen und unterscheiden können, z.B. zur Differenzierung von Analyten zur Differentialdiagnostik, sind dabei gefragt. Im Fokus des Interesses sind vor allem Testsysteme die klein und portabel sind und sich zum Einmalgebrauch eignen.

Antigen/Antikörper-Reaktionen zeichnen sich ganz besonders durch eine sehr hohe Selektivität sowie Sensitivität aus. Die Spezifität dieser Antigen/Antikörper-Reaktionen kann in der Entwicklung neuer Technologien, sogenannter Immunosensoren, genutzt werden. Dabei wird durch den Einsatz entsprechender Transducer das molekulare Bindungsereignis zwischen einem Antikörper und einer nachzuweisenden Substanz (Antigen) in ein makroskopisch messbares Sensorsignal verwandelt. Als nachzuweisende Substanzen können alle bindungsfähigen Moleküle verwendet werden, die mit einem Antikörper koppeln. Unter Antigen wird allgemein ein artfremder Stoff verstanden, der im Körper die Bildung von Antikörpern gegen sich selbst hervorruft.

Ein Allergen ist ein eine Allergie auslösender Stoff, wie z. B. Blütenstaub, Haare, Staub, usw, der eine Allergie bei Menschen hervorrufen kann, die gegen solche Stoffe besonders empfindlich sind. Die Allergie ist somit eine krankhafte Reaktion des Körpers auf bestimmte körperfremde Stoffe.

Ein Antikörper ist ein im Blutserum feststellbarer Schutzstoff (Protein), der als Reaktion auf das Eindringen von Antigenen hin gebildet wird.

Die Antigen-Antikörper-Reaktion bedeutet eine Verbindung eines Antigenes mit dem spezifischen gegen dieses Antigen gerichteten Antikörper zum Antigen-Antikörper-Komplex (Immunokomplex).

Für den Nachweis verschiedener Antigene existieren unterschiedliche Methoden zur Detektion der Antigen/Antiköper-Reaktion. Nachfolgend werden die verschiedenen Ausleseverfahren kurz erläutert:
Piezoelektrische Immunosensoren beruhen auf massensensitiven Schwingquarzen, die als Transducer dienen. Die Änderung der massenabhängigen Resonanzfrequenz des Quarzes gibt Auskunft über die Menge an gebundenem Analyten auf dem Quarz [VI]. Potentiometrische Immunosensoren werten die durch die Antigen/Antikörper-Reaktion entstandene Potentialverschiebung an einer Elektrodenoberfläche aus [III]. Optische Immunosensoren [V; IV]
   a. direkte Messmethoden: z.B. durch Ellipsometrie oder O-berflächenplasmonenresonanz wird aus der Schichtdicken-Zunahme durch Ausbildung des Affinitätskomplexes auf die Menge an gebundenem Analyt geschlossen.
   b: Indirekte Messmethoden: hierbei wird zur Detektion eines messbaren Signals eine Markierung, d.h. eine chemische Modifizierung einer der Immunokomponente benötigt.
      b.1.:Radioimmunoassay ist dieser Marker ein radioaktives Isotop, durch Messung des Isotopenzerfalls wird die Konzentration an gebundener markierter Substanz bestimmt. Häufiger wird jedoch ein optisches Signal erzeugt.
      b.2. Fluoreszenzimmunoassay beispielsweise trägt eine Immunokomponente einen Fluoreszenzmarker, die Intensität der Fluoreszenz führt zum Sensorsignal.
      b.3.Enzymimmunoassays basieren auf der Existenz eines enzymmarkierten Reaktionspartners.

Die Enzyme katalysieren nach Zugabe eines geeigneten Substrats eine Spaltungsreaktion zu einem optisch aktiven Molekül. Die Auslesung des Sensorsignals erfolgt dann entweder photometrisch, d.h. eine Farbreaktion wird detektiert oder durch Chemilumineszenz.

Der Erfindung liegt die Aufgabe zugrunde einen Biosensor zum Nachweis einer ausgewählten Antigen/Antikörper-Reaktion und ein Betriebsverfahren bereitzustellen, womit eine Vielzahl von Analyten mit hoher Empfindlichkeit detektierbar sind.

Die Lösung dieser Aufgabe geschieht jeweils durch die entsprechende Merkmalskombination von Anspruch 1 oder 8. Vorteilhafte Ausgestaltungen sind den Unteransprüchen zu entnehmen.

Der Erfindung liegt die allgemeine Erkenntnis zugrunde, dass
- Anitgen/Antikörper-Reaktionen mit einem enzymgekoppelten Detektionsantikörper markiert werden können,
- spezielle Proteine wie beispielsweise Protein A, G, oder G' eine gerichtete Bindung der Antikörpermoleküle verursacht,
- ein Redoxrecycling durch enzymatische Abspaltung von beispielsweise pAP (para aminophenol) an IDS induziert werden kann,
- zur elektrochemischen Auslesung des Redoxrecycling eine Gegenelektrode und eine Referenzelektrode notwendig sind,

Im Folgenden werden anhand von schematischen, die Erfindung nicht einschränkende Zeichnungen, Ausführungsbeispiele beschrieben:
- Figur 1: zeigt einen schematischen Aufbau des Testsystems basierend auf einer Antigen/Antikörper-Reaktion auf den Goldelektroden des Biochips,
- Figur 2: zeigt Elektrodenvorgänge an den Interdigitalelektroden bei der amperometrischen Auslesereaktion mit p-Aminophenol,
- Figur 3: zeigt einen schematischen Aufbau eines Messplatzes zur Auslesung des Sensorchipsignales,
- Figur 4: zeigt die Schematische Darstellung des verwendeten Sensorchips mit Referenzelektrode, Steckerkontakt für Mikropotentiostaten und Vergrößerung des Siliciumchips mit Interdigitalstrukturen und Gegenelektrode,
- Figur 5: zeigt einen schematischen Aufbau einer Interdigitalstruktur,
- Figur 6: zeigt ein Beispiel eines Sensorsignals mit positiver (Protein A, Maus IgG, Anti-Maus IgG (AP)) und negativer Probe (Protein A, Maus IgG).

Aufbau eines elektronisch auslesbaren Biosensors mit entsprechenden Eigenschaften zum Nachweis einer Antigen-Antikörper-Reaktion:
- Die Siliziumchips, die über eine Leiterplatte mit dem Auslesegerät kontaktiert werden, tragen je nach Layout eine unterschiedliche Anzahl an interdigitalen Elektrodenstrukturen 12, die paarweise angeordnet sind, also immer Anode/Kathode, und je nach Chiplayout einen variablen Durchmesser haben, wobei auf einem Chip immer der gleiche Durchmesser verwendet wird). Die kammartigen Elektrodenfinger der Interdigitalstrukturen weisen dabei immer eine Breite von 1 µm und einen Abstand der Elektrodenpaare von maximal 1,0 µm auf, zudem befindet sich eine Gegenelektrode 11 aus Gold auf dem Chip. Die Ag/AgCl Referenzelektrode 9, gegen welche das Elektrodenpotential eingestellt wird, befindet sich nicht auf dem Chip, sie ist in das Flusssystem integriert und kann optional aber auch on Chip realisiert sein.
- Die Immobilisierung des Antikörpers 5 erfolgt auf den Goldelektroden des Sensorchips. Dies geschieht in Abhängigkeit von der Art des Antikörpers 5 mit Hilfe einer Proteinschicht aus Protein A, G oder G'.
- Das nachzuweisende Antigen 6 , der Analyt, wird mittels Antigen/Antikörper-Kopplung an den auf dem Biochip immobilisierten Antikörper 5 binden.
- Diese Bindung wird mittels eines enzymmarkierten zweiten Antikörpers 7 nachgewiesen.
- Die elektrische Auslesung des Sensorsignals erfolgt mit einem Multichannel Potentiostaten, womit ein konstantes Potential an die Interdigitalelektrodenpaare angelegt wird, nach enzymatischer Freisetzung eines redoxreaktiven Moleküls (wie beispielsweise p-Aminophenol) an der Sensoroberfläche 1.

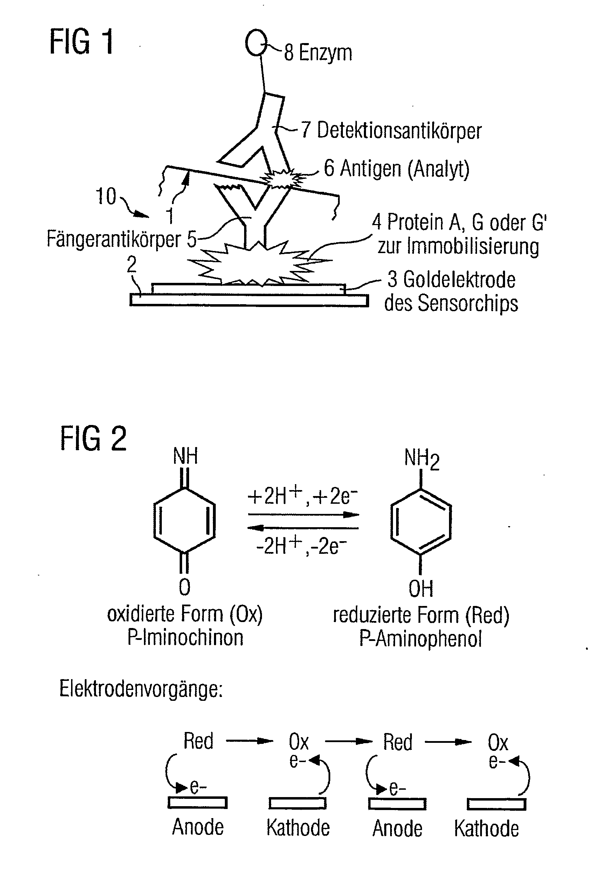

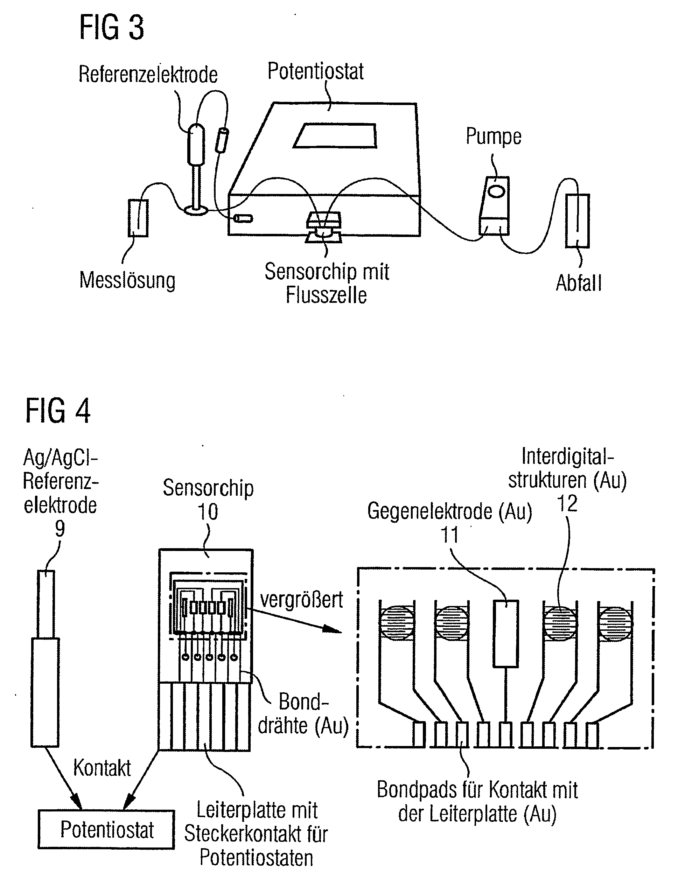

Der Biosensor basiert auf einem Siliziumchip mit Gold-Interdigitalstrukturen, **Fig 4**, im Mikrometerbereich, wobei der Elektrodenpaarabstand kleiner ist als 1 µm ist. Lit [I) und [II].
Durch folgenden Schichtaufbau auf den Gold-Interdigitalstrukturen des Biochips soll der Antigen-Nachweis realisiert werden, **Fig 1**.

### Chipbeschichtung:

**Erster Schritt** ist die Herstellung einer Proteinbasisbeschichtung, die durch Adsorption von z.B. Protein A, G, G'oder L direkt auf der Goldoberfläche der Interdigitalstruktur erfolgt, wobei die Wahl des Proteins abhängig ist von der Art des Fängerantikörpers 5.
**Zweiter Schritt** ist die gerichtete Bindung des Fängerantikörpers 5 an die erste Proteinbasisschicht. Damit wird eine definierte und hoch selektive Chipoberfläche geschaffen.

### Messung zur Detektion des Antigens bzw. des Analyten:

Nach der Kontaktierung der Chipoberfläche 1 mit der zu analysierenden Probe, beispielsweise Wasserprobe, Serum, Lebensmittelextrakt etc., die den entsprechenden Analyten/Antigen beinhaltet, wird das Antigen selektiv an den Fängerantikörper 5 gebunden. Zu den Antigenen zählen alle Moleküle die durch oder an einen Antikörper gebunden werden können; dies können hochmolekulare organische Verbindungen sein, wie Antikörper, aber auch Proteine, Proteinfragmente, Mikroorganismen, Tumormarker, Toxine oder niedermolekulare organische Verbindungen wie Arzneimittel, Pestizide, Antibiotika, Antimykotika oder aromatische Kohlenwasserstoffe.

Das gebundene Antigen 6 ist durch einen Detektionsantikörper 7 markiert.

Der schematische Aufbau des Systems zum Betrieb eines Biosensors basiert auf einer Antigen/Antikörper-Reaktion, die auf den Goldelektroden 3 des Biochips entsprechend **Fig 1** auslesbar ist.

Zur **elektrischen Auslesung** wird der Biochip mit einem Potentiostaten kontaktiert **Fig 2** und das zu dem eingesetzten Enzym passende Substrat z.B. p-Aminophenylphosphat über ein Fluidiksystem, **Fig 2**, zugeführt. Durch das Enzym Alkalische Phosphatase, gekoppelt an den Detektionsantikörper 7 wird das Substrat para aminophenylphosphat zu dem redoxaktiven p-Aminophenol umgesetzt. Bei einer bestimmten angelegten Spannung, wie beispielsweise 350 mV zwischen den Elektroden des Biochips unterliegt das freigesetzte p-Aminophenol einer Reduktion und anschließender Oxidation an den Kathoden und Anoden der Interdigitalelektroden 12 **Fig 2**, wodurch eine Änderung des Sensorsignal, als messbarer Strom im nA Bereich erzeugt wird.
Neben der beschriebenen amperometrischen Auslesemethode unter konstanter Spannung kommt jedoch auch eine Detektion unter Wechselstrom bzw. mittels cyclischer Voltammetrie in Frage.

Elektrodenvorgänge an den Interdigitalelektroden bei der amperometrischen Auslesereaktion mit p-Aminophenol werden in **Fig 2** gezeigt.

Zur Detektion kann auch ein anderer enzymgebundener Detektionsantikörper 7 verwendet werden. Anstelle von p-Aminophenylphosphat muss dann jedoch auch ein anderes"passendes" Substrat eingesetzt werden, welches mit dem verwendeten Enzym reagiert. Weitere passende enzymgebundene Antikörper bzw. deren Substrate sind: β-Galactosidase, gekoppelt an den Detektionsantikörper und p-Aminophenyl-β-D-Galactopyranosid als Substrat.

Wesentliche Vorteile der Erfindung bestehen darin, dass mit Hilfe dieses beispielsweise amperometrischen Antikörper-Biochips durch entsprechende Wahl des Antikörpers eine Vielzahl von Antigenen nachgewiesen werden können.
Gegenüber der herkömmlichen Methoden zeichnet sich das System durch
- seine geringe Größe,
- die geringe Dauer des Tests (<10 min) und
- seine hohe Empfindlichkeit aus.

Aufgrund dieser Eigenschaften und der geringen Produktionskosten des Siliziumchips eignet sich das Messsystem als "Einmal-Analysesystem" für
- medizinische Diagnostik
- Lebensmittelüberwachung
- Umweltüberwachung
- Trinkwasserkontrolle
- Sicherheitstechnik (Detektion von B-Waffen wie Anthrax, Pocken)
- Drogen-/Dopingtests

Es ist normalerweise keine Probenvorbereitung nötig.

Durch Verwendung eines elektrischen Biochip können die Nachteile von optischen Methoden, die vor allem in dem hohen apparativen Aufwand liegen, verringert werden.

### Messaufbau:

### a) Messplatz

Einen schematischer Aufbau des Messplatzes zur Auslesung der Sensorchips zeigt **Fig 3**.

### b) Aufbau des Sensorchip-Systems

Eine schematische Darstellung des verwendeten Sensorchips mit Referenzelektrode 9, Steckerkontakt für Mikropotentiostaten und Vergrößerung des Siliciumchips mit Interdigitalstrukturen 12 und Gegenelektrode 11 zeigt **Fig 4**.

Alternativ zur externen Referenzelektrode kann sich die Ag/AgCl-Elektrode auch auf dem Siliziumchip befinden.

### c) Interdigitale Elektrodenstrukturen

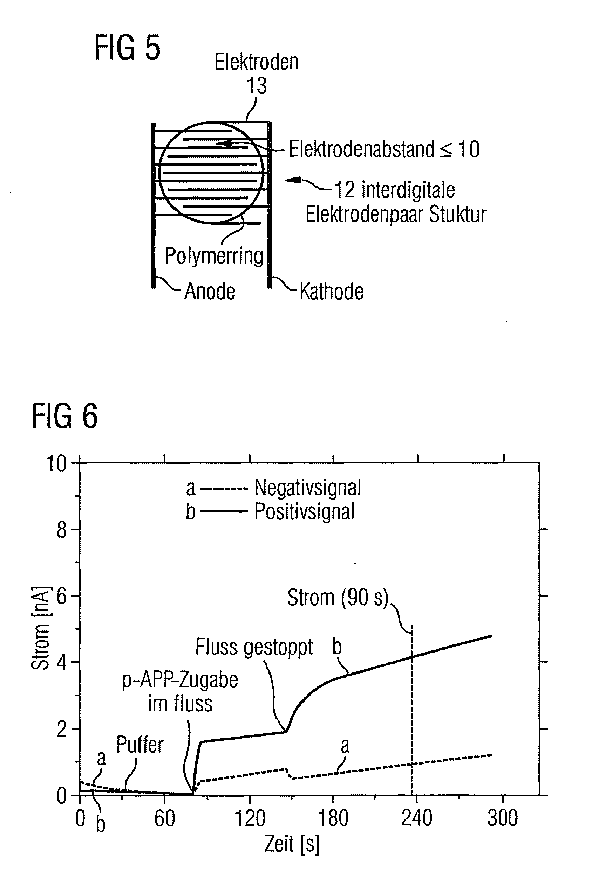

Einen schematischen Aufbau einer Interdigital-Elektrodenstruktur zeigt **Fig 5**.

Am Beispiel eines Maus Immunglobulin G (IgG) als Antikörper und Anti-Maus IgG gekoppelt an Alkalische Phosphatase als Antigen 6 wurde die Funktionsfähigkeit des vorgestellten Messprinzips getestet. Dabei wurden zwei Positionen, entsprechend zweier interdigitaler Elektrodenpaarstrukturen 12, eines Chips parallel beschichtet und ausgelesen. Mittels Protein A wurde Maus IgG auf der Elektrodenoberfläche als Fängerantikörper 5 gebunden. Da Anti-Maus IgG bereits enzymgebunden ist, stellt die Antigen/Antikörper-Reaktion in diesem Fall auch gleichzeitig den Detektionsschritt dar. Anschließend wird der Chip ausgelesen. Hierfür wird der Sensor in die Flusszelle eingebaut, mit dem Multichannel Potentiostaten kontaktiert und zunächst mit Puffer gespült. Nach der Zugabe von p-Aminophenylphosphat wird etwa 30 Sekunden gewartet und dann der Fluss gestoppt, **Fig 6****.**

**Fig 6** zeigt ein Beispiel eines Sensorsignals mit positiver, also vorhandenem Analyten, (Protein A, Maus IgG, Anti-Maus IgG (AP)) und negativer Probe (Protein A, Maus IgG).

**In** **Fig 6** ist zu erkennen, dass bei Zugabe des Substrats p-Aminophenylphosphat (p-APP) an beiden Positionen ein Anstieg des Sensorsignals entsteht, obwohl nur an der Positivposition p-Aminophenol abgespalten wird. Dies kommt dadurch, dass bei eingeschaltetem Fluss an der Positivposition generiertes p-Aminophenol einem aktiven Transport innerhalb der Zelle unterliegt. Aus diesem Grund wird zur Auswertung des Sensorsignals das Verhalten der jeweiligen Positionen bei Flussstop betrachtet. Im Falle einer positiven Signalantwort steigt das Signal bei Flussstop an. Der Grund dafür liegt in der zunehmenden Konzentration an p-Aminophenol an der enzymbelegten Position, welches nicht durch den Fluss abtransportiert wird. Gleichzeitig wird das entstandene p-Aminophenol nicht zu der benachbarten Negativposition transportiert, so dass dort die Konzentration und damit auch das messbare Stromsignal bei Abschalten des Flusses einbricht.

### Literatur:

[I] Hintsche, R. and M. Paeschke (2000). Detektion von Molekülen und Molekülkomplexen. Patentschrift DE 19610115C2. Deutschland.
[II] Hintsche, R., M. Paeschke, et al. (1997). Microbiosensors using electrodes made in Si-technology. Frontiers in Biosensorics 1 - Fundamental Aspects: 267 - 283.
[III]Paeschke, M., F. Dietrich, et al. (1996). "Voltammetric Multichannel Measurements Using Silicon Fabricated Microelectrode Arrays." Electroanalysis 8(10): 891 - 898.
[IV] Lyon, L. A., M. D. Musick, et al. (1999). "Surface plasmon resonance of colloidal Au-modified gold films." Sensors and Actuators B 54: 118 - 124.
[V] Schindler, F. (1992). "Real-Time BIA." BioTec 1: 36-43.
[VI] Uttenthaler, E., C. Kößlinger, et al. (1998). Quartz crystal biosensor for the detection of the African Swine Fever diseas.

## Patentansprüche

1. Biosensor zur Detektion eines Antigenes (6) mittels einer Antigen/Antikörper-Kopplung, der aus folgenden Elementen besteht:
- einem Siliziumsubstrat (2),
- mindestens einer auf dem Siliziumsubstrat (2) aufgebrachten interdigitalen Elektrodenpaarstruktur (12) mit einer Beabstandung der Elektrodenpaare (13) von maximal 1,0 µm,
- einer auf dem Siliziumsubstrat (2) aufgebrachten Gegenelektrode (11),
- einer Referenzelektrode (9),
- einer zumindest die interdigitale Elektrodenstruktur (12) überdeckende ersten Schicht aus Protein (4),
- einer über der ersten Schicht aufgebrachten selektiven zweiten Proteinschicht die einen entsprechend dem zu detektierenden Antigen (6), ausgewählten Fängerantikörper (5) enthält (mit dem das Antigen koppeln kann),
- wobei ein Sensorsignal an der interdigitalen Elektrodenstruktur (12) auslesbar ist, wenn aus einer mit dem Biosensor in Kontakt stehenden zu analysierenden Probe das Antigen (6) an den Fängerantikörper (5) gekoppelt ist und mittels eines enzymmarkierten ebenfalls mit dem Antigen (6) gekoppelten Detektionsantikörpers (7) eine enzymatische Freisetzung eines redoxreaktiven Moleküls an der Sensoroberfläche (1) erfolgt.

2. Biosensor nach Anspruch 1, bei dem die erste Proteinschicht aus den Proteinen A, G oder G' besteht.

3. Biosensor nach einem der vorhergehenden Ansprüche, bei dem zur Selektivitätssteigerung der zweiten Schicht die Fängerantikörper (5) eine gerichte Bindung zum Protein (4) der ersten Schicht aufweisen.

4. Biosensor nach einem der vorhergehenden Ansprüche, bei dem anstelle der amperometrischen Auslesung mittels Redoxrecycling eine Signaldetektion unter Wechselstrom oder cyclischer Voltammetrie erfolgt.

5. Biosensor nach einem der Ansprüche 1 oder 2, der zur Auslesung des Sensorsignales mit einem Potentiostaten gekoppelt ist.

6. Biosensor nach einem der vorhergehenden Ansprüche, bei dem die zu analysierende Probe als Fluid an der Oberfläche (1) des Biosensors über ein Flusssystem bereitgestellt ist.

7. Biosensor nach einem der vorhergehenden Ansprüche, bei dem interdigitale Elektrodenstrukturen (12) und Gegenelektrode (11) aus Gold bestehen.

8. Biosensor nach einem der vorhergehenden Ansprüche, bei dem die Referenzelektrode eine Ag/AgCl - Referenz darstellt.

9. Biosensor nach einem der vorhergehenden Ansprüche, bei dem die Referenzelektrode auf dem eine Referenzelektrode (9) auf dem Biosensor integriert ist.

10. Biosensor nach einem der vorhergehenden Ansprüche, bei dem das Antigen (6) gleichzeitig ein Allergen ist.

11. Biosensor nach einem der vorhergehenden Ansprüche, bei dem das Antigen (6) ein Protein, ein Polypeptid oder Oligopeptid ist.

12. Biosensor .nach einem der Ansprüche 1 bis 10, bei dem das Antigen ein Mikroorganismus wie ein Bakterium oder ein Virus ist.

13. Biosensor nach einem der Ansprüche 1 bis 11, bei dem das Antigen eine organische Verbindung wie ein Toxin, Arzneimittel, Pestizid, Anthrax, Antibiotikum oder aromatischer Kohlenwasserstoff ist.

14. Verfahren zum Betrieb eines Biosensors zur Detektion eines Antigenes (6) mittels einer Antigen/Antikörper-Kopplung, das folgende Schritte aufweist:
- Beschichtung eines auf einem Siliziumchip aufgebauten Biosensors mit einer Proteinbasisbeschichtung mit einem Protein A, G oder G' bei gleichzeitiger Überdeckung von interdigitalen Elektrodenpaarstrukturen (12) auf der Oberfläche des Siliziumschips,
- Herstellung einer weiteren Schicht auf der Proteinbasisbeschichtung welche einen Fängerantikörper (5) enthält, welcher derart ausgewählt ist, dass dieser mit dem gesuchten Antigen (6) koppeln kann,
- Kontaktierung der Sensoroberfläche (1) mit einem zu analysierenden Fluid, wobei ein im Fluid enthaltenes Antigen selektiv an den Antikörper der obersten Schicht gebunden werden kann,
- Markierung des Antigenes (6) durch einen Detektionsantikörpers (7), der mit einem Enzym gekoppelt ist und der gleichzeitig mit dem Antigen (6) koppelt,
- Auslesung eines Sensorsignales mittels eines Potentiostaten durch Redoxrecycling, wobei der enzymgebundene Detektionsantikörper (7) eine enzymatische Freisetzung eines redoxreaktiven Moleküles an der Sensoroberfläche bewirkt und sich Gegeneelektrode und Referenzelektrode im gleichen Flusssystem wie die Sensoroberfläche befinden, und sich die Gegenelektrode auf dem Chip befindet.

## Claims

1. Biosensor for detection of an antigen (6) by means of an antigen/antibody coupling, consisting of the following elements:
- A silicon substrate (2),
- At least one interdigital electrode pair structure (12) accommodated on the silicon substrate (2) with a spacing between the electrode pairs (13) of maximum 1.0 µm,
- A counter-electrode (11) accommodated on the silicon substrate (2),
- A reference electrode (9),
- A first layer made of protein (4) at least covering over the interdigital electrode structure (12),
- A selective second protein layer applied over the first layer which contains a selected capture antibody (5) corresponding to the detecting antigen (6) and which can couple to the antigen,
- with a sensor signal being able to be read out at the interdigital electrode structure (12) if, from a sample to be analyzed which is in contact with the biosensor, the antigen (6) is coupled to the capture antibody (5) and by means of an enzyme-marked detection antibody (7) also coupled to the antigen, an enzymatic release of a redox-reactive molecule on the sensor surface (1) occurs.

2. Biosensor according to claim 1, in which the first protein layer consists of the proteins A, G or G'.

3. Biosensor according to one of the previous claims, in which for increasing selectivity of the second layer the capture antibodies (5) feature a directed binding to the protein (4) of the first layer.

4. Biosensor according to one of the previous claims, in which, instead of the amperometric readout by means of redox recycling, a signal is detected using alternating current or cyclic voltammetry.

5. Biosensor according to one of the claims 1 or 2, which is coupled with a potentiostat for readout of the sensor signal.

6. Biosensor according to one of the previous claims, in which the sample to be analyzed is provided as fluid on the surface (1) of the biosensor via a flow system.

7. Biosensor according to one of the previous claims, in which interdigital electrode structures (12) and counter-electrode (11) are made of gold.

8. Biosensor according to one of the previous claims, in which the reference electrode represents an Ag/AgCl reference.

9. Biosensor according to one of the previous claims, in which the reference electrode is integrated onto the one reference electrode (9) on the biosensor.

10. Biosensor according to one of the previous claims, in which the antigen (6) is simultaneously an allergen.

11. Biosensor according to one of the previous claims, in which the antigen (6) is a protein, a polypeptide or oligopeptide.

12. Biosensor according to one of the claims 1 to 10, in which the antigen is a microorganism such as a bacterium or a virus.

13. Biosensor according to one of the claims 1 to 11, in which the antigen is an organic compound such as a toxin, medicine, pesticide, anthrax, antibiotic or aromatic hydrocarbon.

14. Method for operation of a biosensor for detection of an antigen (6) by means of an antigen/antibody coupling, which features the following steps:
- Coating of a biosensor constructed on a silicon chip with a protein base coating with a protein A, G or G' with simultaneous covering of interdigital electrode pair structures (12) on the surface of the silicon chip,
- Fabrication of a further layer on the protein coating which contains a capture antibody (5) which is selected so that it can coupled with the antigen (6) sought,
- Contacting of the sensor surface (1) with a fluid to be analyzed, with an antigen contained in the fluid being able to be bound selectively to the antibodies of the uppermost layer,
- Marking of the antigen (6) by a detection antibody (7) which is coupled with an enzyme and which simultaneously couples with the antigen (6),
- Readout of a sensor signal by means of a potentiostat through redox recycling, with the enzyme-bound detection antibody (7) causing an enzymatic release of a redox-reactive molecule on the sensor surface and counter-electrode and reference electrode being located in the same flow system as the sensor surface and the counter-electrode being located on the chip.

## Revendications

1. Biocapteur pour la détection d'un antigène ( 6 ) au moyen d'un couplage antigène/anticorps, qui est constitué des éléments suivantes :
- un substrat ( 2 ) de silicium,
- au moins une structure ( 12 ) de paires d'électrodes interdigitées déposées sur le substrat de silicium avec un intervalle de paires ( 13 ) d'électrodes de 1,0 µm au maximum,
- une contre-électrode ( 11) déposée sur le substrat ( 2 ) de silicium,
- une électrode de référence ( 9 ),
- une première couche de protéine ( 4 ) recouvrant au moins la structure ( 12 ) d'électrodes interdigitées,
- une deuxième couche de protéine sélective déposée sur la première couche et contenant un anticorps ( 5 ) de capture choisi en fonction de l'antigène ( 6 ) à détecter ( avec lequel l'antigène peut se coupler ),
- dans lequel un signal de capteur peut être lu sur la structure ( 2 ) d'électrodes interdigitées lorsque, à partir d'un échantillon à analyser en contact avec le biocapteur, l'antigène ( 6 ) est couplé sur l'anticorps ( 5 ) de capture et il se produit, au moyen d'un anticorps ( 7 ) de détection marqué par un enzyme et couplé également à l'antigène ( 6 ), une libération enzymatique d'une molécule réactive de façon rédox à la surface ( 1 ) du capteur.

2. Biocapteur suivant la revendication 1, dans lequel la première couche de protéine est constituée des protéines A, G ou G'.

3. Biocapteur suivant l'une des revendications précédentes, dans lequel, pour augmenter la sélectivité de la deuxième couche, les anticorps ( 5 ) de capture ont une liaison dirigée vers la protéine ( 4 ) de la première couche.

4. Biocapteur suivant l'une des revendications précédentes, dans lequel, au lieu de la lecture ampérométrique au moyen d'un recyclage rédox, il se produit une détection de signal en courant alternatif ou en voltamétrie cyclique.

5. Biocapteur suivant la revendication 1 ou 2, qui est couplée à un potentiostat pour la lecture du signal du capteur.

6. Biocapteur suivant l'une des revendications précédentes, dans lequel on prépare l'échantillon à analyser sous la forme d'un fluide à la surface ( 1 ) du biocapteur par un système de flux.

7. Biocapteur suivant l'une des revendications précédentes, dans lequel des structures ( 12 ) d'électrodes interdigitées et des contre-électrodes ( 11 ) sont en or.

8. Biocapteur suivant l'une des revendications précédentes, dans lequel l'électrode de référence constitue une référence Ag/AgCl.

9. Biocapteur suivant l'une des revendications précédentes, dans lequel l'électrode de référence est intégrée sur une électrode ( 9 ) de référence sur le biocapteur.

10. Biocapteur suivant l'une des revendications précédentes, dans lequel l'antigène ( 6 ) est en même temps un allergène.

11. Biocapteur suivant l'une des revendications précédentes, dans lequel l'antigène ( 6 ) est une protéine, un polypeptide ou un oligopeptide.

12. Biocapteur suivant l'une des revendications 1 à 10, dans lequel l'antigène est un micro-organisme ou une bactérie ou un virus.

13. Biocapteur suivant l'une des revendications 1 à 11, dans lequel l'antigène est un composé organique comme une toxine, un médicament, un pesticide, un anthrax, un antibiotique ou un hydrocarbure aromatique.

14. Procédé pour faire fonctionner un biocapteur pour la détection d'un antigène ( 6 ) au moyen d'un couplage antigène/anticorps, qui a les stades suivantes :
- on revêt un biocapteur constitué d'une puce de silicium, d'un revêtement de base en protéine ayant une protéine A, G ou G', avec recouvrement simultané de structures ( 12 ) de paires d'électrodes interdigitées à la surface de la puce en silicium,
- on produit une autre couche de revêtement de base en protéine, qui contient un anticorps ( 5 ) de capture, lequel est choisi de façon à pouvoir se coupler avec l'antigène recherché,
- on met la surface ( 1 ) du capteur en contact avec un fluide à analyser, un antigène contenu dans le fluide pouvant se fixer sélectivement à l'anticorps de la couche la plus haute,
- on marque l'antigène ( 6 ) par un anticorps ( 7 ) de détection, qui est couplé à un enzyme et qui se couple en même temps à l'antigène ( 6 ),
- on lit un signal du capteur au moyen d'un potentiostat par recyclage rédox, l'anticorps ( 7 ) de détection fixé à un enzyme provoquant une libération enzymatique d'une molécule réactive d'une façon rédox à la surface du capteur et une contre-électrode et une électrode de référence se trouvant dans le même système de flux que la surface du capteur et la contre-électrode se trouvant sur la puce.
